(19) Europäisches Patentamt
European Patent Office
Office européen des brevets

(11) Numéro de publication: **0 226 475**
**A1**

(12) # DEMANDE DE BREVET EUROPEEN

(21) Numéro de dépôt: 86401631.6

(22) Date de dépôt: 22.07.86

(51) Int. Cl.⁴: C 07 C 93/06
A 61 K 31/13

(30) Priorité: 22.07.85 FR 8511151
16.10.85 FR 8515449

(43) Date de publication de la demande:
24.06.87 Bulletin 87/26

(84) Etats contractants désignés:
AT BE CH DE FR GB IT LI LU NL SE

(71) Demandeur: Albert ROLLAND S.A. Société dite
49, Rue St-André-des-Arts
F-75006 Paris(FR)

(72) Inventeur: Labaune, Jean-Pierre
10 rue des Grands Cèdres
F-77310 Ponthierry-Moulignon(FR)

(74) Mandataire: Burtin, Jean-François
5 Bis, rue Parmentier
F-92200 Neuilly S/Seine(FR)

(54) **Nouveaux dérivés de la diphénoxyethylamine, leur procédé d'obtention et les compositions pharmaceutiques en renfermant.**

(57) L'invention se rapport au domaine de la chimie et notamment de la chimie pharmaceutique.

Elle a pour objet les dérivés N-alcoylés de la 2,2-biphenoxyethylamine de formule générale I

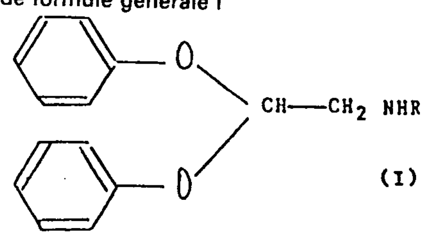

(I)

dans laquelle R représente un radical alcoyle inférieur sous forme libre ou salifiée.

Ces composés servent comme principes actifs de compositions pharmaceutiques à visée antidépressive.

EP 0 226 475 A1

# NOUVEAUX DERIVES DE LA DIPHENOXYETHYLAMINE, LEUR PROCEDE D'OBTENTION ET LES COMPOSITIONS PHARMACEUTIQUES EN RENFERMANT

La présente invention a pour objet de nouveaux dérivés de la diphenoxyethylamine et leur procédé d'obtention.

Elle a plus particulièrement pour objet des dérivés N-alcoylés de la 2,2-bis phenoxyethylamine.

La présente invention concerne spécifiquement les dérivés N-alcoylés de la 2,2-bis phenoxyethylamine de formule générale I

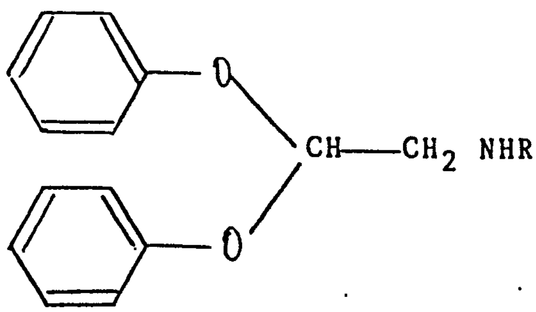

(I)

dans laquelle R représente un radical alcoyle inférieur ayant de 1 à 4 atomes de carbone en chaine droite ou ramifiée.

L'invention se rapport également aux sels d'addition des composés de formule générale I avec un acide minéral ou organique, de préférence un acide thérapeutiquement compatible.

Parmi les sels d'addition utilisables, on pourra citer les bromhydrates, chlorhydrates, codhydrates, les sulfates, les nitrates, les phosphates, les hypophosphates, les thiosulfates ou les sulfites ; les formiates, les acétates, les benzoates, les naphtoates, les nicotinates, les isonicotinates, les trimethoxy 3,4,5-benzoates, les syringoates, les O-carbethoxy-syringoates, les benzenesulfonates, les methanesulfonates, les isethionates, les p. toluenesulfonates, les naphtalene sulfonates, les tratrates, les pyruvates, les citrates, les maléates,

les fumarates, les itaconates, les citraconates, les malonates, les trationates, les lactobionates, les glucose 1-phosphates et les glucoses 1,6-diphosphates.

Les sels d'acides qui ne peuvent être utilisés en thérapeutique comme les iodates, les periodates, les ferrocyanures ou les strychnates, peuvent servir comme moyens d'identification ou de purification. Ils seront ensuite reconvertis en base de formule générale (I).

Parmi les composés de formule générale I, R symbolise de préférence un radical methyle, ethyle ou isopropyle. Il peut aussi représenter un terbutyle ou un sec butyle.

L'invention concerne aussi un procédé d'obtention des composés de formule générale I ainsi que de leurs sels qui consiste à transformer l'acide 2,2-bis phenoxy acetique en un dérivé fonctionnel, faire réagir celui-ci avec une amine primaire de formule générale II

$$R\ NH_2 \qquad (II)$$

dans laquelle R est défini comme précédemment pour former un amide de formule III

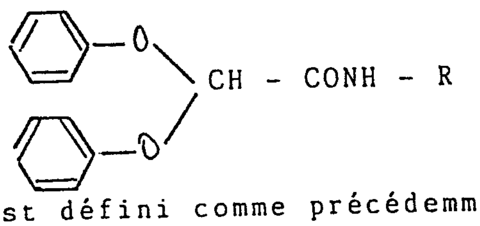

dans laquelle R est défini comme précédemment, puis à réduire par un hydrure mixte de métal alcalin, l'amide formé en un composé de formule générale I. Le composé de formule générale I pourra en outre être salifié par addition d'un acide minéral ou organique.

En variante du procédé défini ci-dessus, il est possible également de partir d'une amine tertiaire telle que la Medifoxamine et de la soumettre à une desalcoylation à l'aide d'un agent quaternisant tel qu'un halogenure de cyanogène ou un chloroformiate d'alcoyle. Dans ce cas, on obtient un composé

de formule I dans laquelle R est un radical methyle.

Dans un mode d'exécution préféré du procédé selon l'invention

- le dérivé fonctionnel de l'acide 2,2-bis phenoxyacetique est un halogenure d'acide, un anhydride d'acide ou un anhydride mixte formé par action d'un agent acylant, comme une carbodiimide, le carbonyl bis imidazole ou un pyrophosphate d'alcoyle
- la formation de l'amide de formule III est effectuée dans un solvant inerte comme, par exemple, un carbure aromatique
- la réduction de l'amide de formule III est effectuée par action d'un hydrure d'aluminium et de métal alcalin comme, par exemple, l'aluminohydrure de lithium, le terbutoxy aluminohydrure de lithium, ou l'isopropylaminohydrure de lithium.

On peut également obtenir un composé de formule générale I à partir d'un ester sulfonique du 2,2-bis phenoxy ethanol tel que le mesylate ou le p.toluènesulfonate, par action d'un amine primaire de formule générale II

$$RNH_2$$

dans un solvant à point d'ébullition élevé.

L'invention concerne aussi les compositions pharmaceutiques renfermant, à titre de principe actif, au moins un composé de formule générale I ou un de ses sels d'addition avec un acide minéral ou organique en mélange avec un excipient ou un véhicule inerte, non toxique, pharmaceutiquement-acceptable.

Les excipients, ou véhicules qui conviennent, sont ceux adaptés pour l'administration par voie parentérale, buccale, percutanée ou rectale.

Les compositions pharmaceutiques se présentent sous forme de comprimés nus ou enrobés, de dragées, de pilules, de sachets, de poudres aromatisées ou non, de solutions ou suspensions

buvables, de solutés injectables répartis en ampoules, en flacons multi-doses ou de seringues auto-injectables, de préparations lyophilisées, de suppositoires ou de préparations dans un solvant polaire pour administration percutanée.

Les compositions pharmaceutiques renferment de 0,025 g à 0,250 g de composé de formule générale I ou d'un de ses sels à titre de principe actif et de préférence de 0,05 g à 0,20 g.

La posologie journalière en composé de formule générale I ou d'un de ses sels variera en fonction du poids du sujet, de l'âge du sujet, de l'indication thérapeutique et de la gravité du cas. En règle générale, la posologie journalière s'échelonnera chez l'adulte de 0,05 g à 0,150 g. . Elle peut être considérablement modulée en fonction des besoins thérapeutiques.

Les composés de formule générale I et leurs sels trouvent un emploi en thérapeutique comme agents anti-dépresseurs. Ils conviennent de ce fait pour l'amélioration ou la guérison des états obsessionnels, des psychoses maniaco-évolutives, des psychoses réactionnelles, des phénomènes de sevrage.

Ils se différencient nettement de la Medifoxamine ou d'autres composés similaires par leur attitude à favoriser la recapture de la noradrénaline et de diminuer celle de la tryptamine Ils ont aussi la possibilité - tout au moins sur des tests pratiqués sur des animaux - de recharger les granules neuronaux pré-synaptiques en substances adrenergiques, et ainsi de restituer à ceux-ci un potentiel d'action normal.

Les exemples suivants illustrent l'invention. Ils ne la limitent en aucune façon.

EXEMPLE I

2,2-bis phenoxy N-methylaminoethane

a) - Ester méthylique de l'acide bromo-2-phénoxy-acétique :

L'ester méthylique de l'acide phénoxy acétique est bromé selon la méthode décrite par J.L. COLIN et LOUBINOUX (Synthesis 568, 1983).

Un mélange d'ester (20 g ; 0,12 mole), de N-bromosuccinide (25,6 g ; 0,14 mole) et de traces (100 mg) de péroxyde de benzoyle, est mis à ébullition dans 500 ml de tétrachlorure de carbone. L'évolution de la réaction est suivie en CCM. avec le mélange acétate d'éthyle 30 ; hexane 70 comme éluant. Après 1 heure de reflux, la réaction est terminée. Après filtration et évaporation, on recueille une huile suffisamment pure pour être utilisée sans purification.

I.R. : C O = 1770 $cm_{-1}$

R.M.N. : 3,8 ppm s($CH_3$) ; 6,4 ppm s(CH) ;
7,2 ppm m(aromatiques)

b) - Acide 2,2-bis phenoxy acétique :

L'ester bromé brut dissous dans 200 ml de toluène contenant 2 g de bromure de tétrabutylammonium (0,0062 mole),est ajouté à une solution aqueuse de soude à 50% (70 ml) contenant 12,42 g de phénol (0,132 mole). Le mélange est agité durant 1 nuit. La phase aqueuse est acidifiée et extraite à l'éther. La phase organique est lavée, séchée et évaporée. L'acide 2,2-bis phénoxy acétique est purifié par chromatographie sur colonne avec $CH_2Cl_2$/MeOH 95/5 comme éluant.

I.R. : C O = 1750 $cm^{-1}$

R.M.N. : 5,9 ppm s(CH) ; 7 ppm m(aromatiques) ;
8,8 ppm s(OH

c) - <u>2,2-bis phénoxy N-methyl-acétamide</u> :

A 3 g d'acide précédent, dans 30 ml de toluène sec, on ajoute 1,5 eq de chlorure d'oxalyle. On porte à 40° C durant 2 heures. L'évolution de la réaction est suivie par I.R. Le chlorure d'acide obtenu, très instable, est utilisé directement. On fait passer un courant de monométhyl amine pendant 1/2 heure. L'agitation est maintenue 10 heures à température ambiante. Après traitement acido-basique, on obtient l'amide d'une pureté satisfaisante.

I.R. : C O = 1670 $cm_{-1}$
R.M.N. : 2,75 ppm d($CH_3$) ; 5,7 ppm s(CH) ;
6,9 ppm n(aromatiques) ; 6,8 ppm (NH)

d) - <u>2,2-bis phénoxy N-methyl-amino-éthane</u> :

A une suspension de 0,4 g (2,8 eq) d'hydrure de lithium et d'aluminium dans 30 ml d'éther sec, on ajoute 1 g d'amide dans 50 ml d'éther à 0° C. Après 1/2 heure, on laisse revenir à température ambiante et on maintient l'agitation 12 heures. Après traitement acido-basique, on récupère l'amine correspondante.

I.R. : NH O = 3300 $cm^{-1}$
R.M.N. : 2,7 ppm (pic large : couplage avec NH,$CH_3$) ;
3,4 ppm (pic large : $CH_2$) ; 6,4 ppm t$(CH)^3$ ;
7 ppm m(aromatiques) ; 9,8 ppm (pic large : NH)

Celle-ci est convertie en chlorhydrate par dissolution de la base dans l'éthanol et saturation de la solution ethanolique par un courant d'acide chlorhydrique gazeux. Le chlorhydrate qui précipite est séparé, lavé à l'éthanol et sèché sous vide.

On prépare d'une manière similaire le maléate ou le fumarate de 2,2-bis phénoxy N-methylamineéthane par addition d'une solution éthanolique d'acide fumarique ou maléique.

EXEMPLE II

2,2-bis phénoxy N-isopropylamino éthane

En opérant comme stade c) de l'exemple I au départ de l'acide 2,2-bis phénoxy acétique et d'isopropylamine, on obtient la 2,2-bis phénoxy N-isopropyl acétamide.

Celle-ci est réduite par l'hydrure de lithium et d'aluminium en opérant selon le mode opératoire du stade d) de l'exemple I.

Le 2,2-bis-phénoxy N-isopropylamino éthane est un produit huileux que l'on convertit en chlorhydrate par action de l'acide chlorhydrique en solvant organique.

EXEMPLE III

Etude pharmacologique des composés selon l'invention.

La vérification des propriétés anti-dépressives a été effectuée selon les tests pharmacologiques classiques :

- antagonisme vis-à-vis de l'hypothermie provoquée par injection de Reserpine
- Antagonisme vis-à-vis de l'hypothermie provoquée par injection d'Apomorphine
- Accroissement de la toxicité de groupe provoquée par injection de Yohimbine.

TABLEAU I

| PRODUIT | STRUCTURE CHIMIQUE | TOXICITÉ AIGUË SOURIS P.O. | HYPOTHERMIE RÉSERPINE 100 P.O. | HYPOTHERMIE APOMORPHINE 50 P.O. | HYPOTHERMIE APOMORPHINE 100 P.O. | TOXICITÉ YOHIMBINE 100 P.O. | TEST DU DÉSESPOIR SOURIS 100 P.O. | CHIEN NA | CHIEN TYR. |
|---|---|---|---|---|---|---|---|---|---|
| MEDIFOXAMINE | 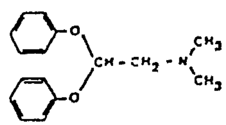 | 600 0/10<br>700 3/10<br>800 5/10 | + 2°5 * | + 1°1 * | + 0°9 * | 5/10 | 60% * | ↑ | ↓ |
| 2,2-bis phénoxy N-méthyl amino éthane | 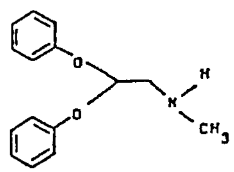 | 200 0/5<br>350 3/5<br>500 5/5 | + 2° * | + 0°7 * | ST | 6/10<br>ST | 50 P.O.<br>52% * | ↑↑ | ↓↓ |

- Antagonisme vis-à-vis du test du desespoir chez la souris

Les composés selon l'invention administrés par voie buccale à la dose de 50 ou 100 mg/kg manifestent, sur ces tests, une activité au moins comparable à celle de la Medifoxamine prise comme substance de référence.

En outre, sur le test du desespoir chez la souris, les composés selon l'invention sont pratiquement aussi actifs à 50 mg/kg per os que la Medifoxamine à 100 mg/kg.

Le Tableau I résume les principaux résultats pharmacologiques obtenus.

EXEMPLE IV

Recherche d'une dose moyenne léthale.

La dose léthale moyenne chez la souris a été recherchée en administrant à des lots de 5 souris, per os, des doses croissantes des composés selon l'invention. Une dose moyenne approchée a été calculée graphiquement par la méthode de Tainter et Miller.

On constate qu'à 200 mg/kg, il n'y a pas de mortalité, à 350 mg/kg, trois animaux sur cinq meurent et qu'à 500 mg/kg, tous les animaux meurent. La $DL_{50}$ se situe donc vers 300 mg/kg.

EXEMPLE V

Etude biologique des composés selon l'invention.

Activité sur les tests d'inhibition ou de recapture des principaux médiateurs adrénergiques.

Les résultats obtenus en comparaison avec ceux des antidépresseurs classiques sont rassemblés dans le tableau II :

TABLEAU II

| PRODUITS | UPTAKE "IN VITRO" $IC_{50}$ M | | | UPTAKE "EX VIVO" % d'inhibition | | |
|---|---|---|---|---|---|---|
| | NAd | 5-HT | DA | NAd | 5-HT | DA |
| DESIPRAMINE | $1,3 \times 10^{-8}$ | | | - 51 % (25 mg/kg) | | |
| IMIPRAMINE | | $5,7 \times 10^{-7}$ | | | | |
| DOPAMINE | | | $8,0 \times 10^{-7}$ | | | |
| MEDIFOXAMINE | $1,9 \times 10^{-6}$ | $8,3 \times 10^{-5}$ | $8,3 \times 10^{-6}$ | inactif | inactif | inactif |
| 2,2-bis-phénoxy N-méthyl amino éthane | $2,0 \times 10^{-7}$ | $1,5 \times 10^{-6}$ | $3,0 \times 10^{-6}$ | - 23 % (100 mg/kg) | - 11 % N.S. | inactif |

Nad = Nor adrénaline

5HT = Sérotonine

DA = Dopamine

EXEMPLE VI

Comprimés à 0,250 g de 2,2-bis-phénoxy N-méthylaminoéthane sous forme de chlorhydrate :

| | |
|---|---|
| Principe actif | 2834 g 1 |
| Sulfate de Calcium | 1510 g |
| Cellulose microcristalline | 325 g |
| Gomme arabique | 16 g |
| Ethyl cellulose | 24 g |
| Povidone K 15 | 30 g |
| Talc | 65 g |

pour 10.000 comprimés d'un poids moyen de 0,48 g.

REVENDICATIONS

L'invention a pour objet :

1°) Les 2,2-bis phénoxyéthylamines de formule générale I

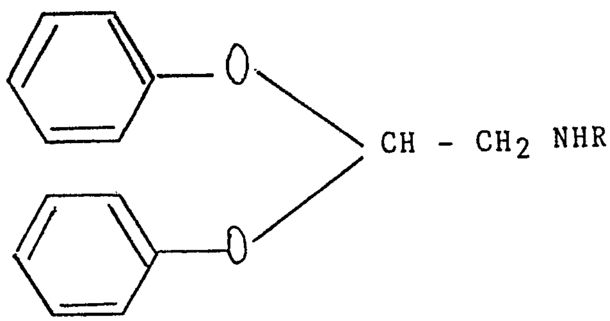

dans laquelle R représente un radical alcoyle inférieur ayant de 1 à 4 atomes de carbone.

2°) Les sels d'addition des composés de formule générale I avec un acide minéral ou organique.

3°) Un composé selon l'une des revendications 1°) ou 2°), à savoir le 2,2-bis phénoxy N-méthylamino éthane et ses sels.

4°) Un composé selon l'une des revendications 1°) ou 2°), à savoir le 2,2-bis phénoxy N-isopropylamino éthane et ses sels.

5°) Un procédé d'obtention des composés selon la revendication 1°) ou la revendication 2°) dans lequel on forme un dérivé fonctionnel de l'acide 2,2-bis phénoxy acétique, condense celui-ci avec une amine primiaire de formule II $RNH_2$

dans laquelle R est défini comme précédemment, puis réduit l'amide secondaire de formule générale III

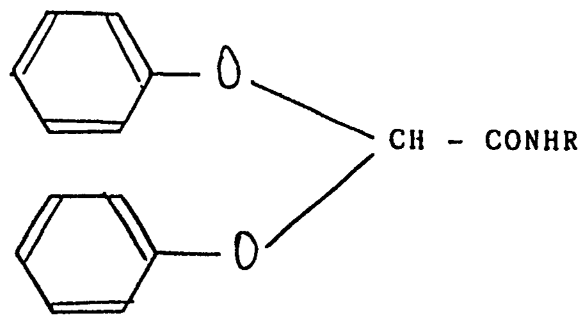

dans laquelle R est défini comme précédemment

par un hydrure mixte de métal alcalin, pour obtenir l'éthylamine désirée de formule générale I que l'on peut, si désiré, salifier par addition d'un acide minéral ou organique.

6°) Les compositions pharmaceutiques renfermant à titre de principe actif au moins un composé selon la revendication 1°) ou la revendication 2°) en association ou en mélange avec un excipient ou un véhicule inerte non-toxique, pharmaceutiquement-acceptable.

7°) Une composition pharmaceutique selon la revendication 6°) dans laquelle l'excipient ou le véhicule est un de ceux appropriés pour l'administration par voie parentérale, buccale, rectale ou percutanée.

8°) Une composition pharmaceutique selon la revendication 6°) dans laquelle la quantité des principes actifs est comprise entre 25 et 250 mg par prise unitaire.

Office européen des brevets

# RAPPORT DE RECHERCHE EUROPEENNE

0226475
Numero de la demande

EP 86 40 1631

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| X | FR-M- 5 498 (LABORATOIRES GERDA)<br>* En entier *<br>----- | 1-8 | C 07 C 93/06<br>A 61 K 31/13 |
| | | | DOMAINES TECHNIQUES RECHERCHES (Int. Cl.4) |
| | | | C 07 C 93/00<br>A 61 K 31/00 |

Le present rapport de recherche a été établi pour toutes les revendications

| Lieu de la recherche | Date d achèvement de la recherche | Examinateur |
|---|---|---|
| LA HAYE | 12-01-1987 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l invention
E : document de brevet antérieur, mais publié à la date de dépôt ou après cette date
D : cité dans la demande
L : cité pour d'autres raisons

& : membre de la même famille, document correspondant

OEB Form 1503 03 82